# EUROPEAN PATENT APPLICATION

(11) **EP 4 834 930 A1**
(43) Date of publication of application: **04.11.2026**
(21) Application number: 24910738.4
(22) Date of filing: 12.12.2024
(51) Int. Cl.: C07K 16/12, C12N 5/20, A61K 39/40, A61P 31/04, G01N 33/577, G01N 33/569, C12R 1/91

(54) **MONOCLONAL ANTIBODY AGAINST CAPSULAR POLYSACCHARIDE OF KLEBSIELLA PNEUMONIAE SEROTYPE K64, HYBRIDOMA CELL LINE THEREOF AND USE THEREOF**

(30) Priority: 27.12.2023 CN 202311815637; 28.11.2024 CN 202411721463
(71) Applicant: Shanghai Bofan Biotherapeutics Co., Ltd., Shanghai 201315 (CN)
(72) Inventor: HE, Ping, Shanghai 201315 (CN); ZHU, Xianchao, Shanghai 201315 (CN); CHEN, Huagen, Shanghai 201315 (CN); MA, Ruijing, Shanghai 201315 (CN); LI, Ying, Shanghai 201315 (CN)
(74) Representative: Bjerkén Hynell KB
(86) International application number: PCT/CN2024/138668
(87) International publication number: WO 2025/139807

(57) **Abstract**

A monoclonal antibody against the capsular polysaccharide of *Klebsiella pneumoniae* serotype K64, a hybridoma cell line thereof and the use thereof. The sequence information of the monoclonal antibody against the capsular polysaccharide of *Klebsiella pneumoniae* serotype K64 is as set forth in SEQ NO: 1-SEQ NO: 56, and the accession numbers of the hybridoma cell lines are CCTCC: C2023365, CCTCC: C2023366, CCTCC: C2023367, CCTCC: C2024340, CCTCC: C2024341, CCTCC: C2024342 and CCTCC: C2024343, respectively. The provided monoclonal antibody against the capsular polysaccharide of *Klebsiella pneumoniae* can specifically bind to the capsular polysaccharide of *Klebsiella pneumoniae,* which is beneficial for the typing of *Klebsiella pneumoniae* in clinical practice. Both in vivo and in vitro animal experiments have demonstrated that the monoclonal antibody against the capsular polysaccharide of *Klebsiella pneumoniae* has excellent effects in preventing and treating infections caused by *Klebsiella pneumoniae* serotype K64, which provides a way to solve the problems of *Klebsiella pneumoniae* infections and multi-drug resistance, has significant significance in the prevention, diagnosis and treatment of *Klebsiella pneumoniae* infections, and has an important value for the development of new-generation drugs against *Klebsiella pneumoniae.*

## Description

### TECHNICAL FIELD

This application belongs to the technical field of medicine and, in particular, relates to a monoclonal antibody directed to a K64-type *Klebsiella pneumoniae* capsular polysaccharide, along with its hybridoma cell line and applications.

### BACKGROUND

*K. pneumoniae* is a Gram-negative bacterium with a capsular glycocalyx. It mainly colonizes the digestive and respiratory tracts. It can cause lung, urinary tract, and blood infections when the body's immune system is compromised. It is also one of the most common bacteria to cause respiratory tract infections.

Infections caused by *K. pneumoniae* have become one of the serious public health problems worldwide. According to the statistics, the mortality rate of the *K. pneumoniae* infections is extremely high. Mortality rates for blood infections caused by *K. pneumoniae* range from 20% to 30%, while the mortality rates for *K. pneumoniae* bacteremia complicated by pneumonia can exceed 50%. Treatment for *K. pneumoniae* mainly relies on antibiotics. However, in recent years, *K. pneumoniae* producing carbapenemases has become resistant to almost all available β-lactam drugs (including carbapenems) due to the widespread use of various antibacterial drugs. The proportion of carbapenem-resistant *K. pneumoniae* (CRKP) has increased dramatically worldwide over the last 10 years. A capsular polysaccharide is a key virulence factor of *K. pneumoniae* and an important protective antigen for treating or preventing *K. pneumoniae* infections. Based on differences in the structure of the capsular polysaccharide, *K. pneumoniae* can be categorized into at least 77 serotypes. The capsular polysaccharide is also referred to as a K antigen or a K type. Different K types exhibit different drug resistance spectra and are distributed differently in geographical locations and in a clinical context. They may also have different epidemic patterns in nosocomial infections. Among them, K64-type *K*. *pneumoniae* is commonly implicated in clinical infections, and the vast majority of these are CRKP strains. Current antibiotic treatment for K64 CRKP strains poses significant challenges in clinical practice.

Consequently, a highly specific, high-affinity monoclonal antibody directed to the K64 capsular polysaccharide is needed to advance the prevention and control of drug-resistant *K. pneumoniae* and to enable its clinical application.

### BRIEF SUMMARY

This application provides a monoclonal antibody directed to the capsular polysaccharide of K64-type *K. pneumoniae,* for solving the aforementioned technical problems. The antibody comprises a light chain variable region and a heavy chain variable region; wherein the light chain variable region has an amino acid sequence as shown in SEQ ID NO: 1, or a sequence with at least 70%, at least 75%, at least 80%, at least 85%, at least 90%, at least 95%, at least 96%, at least 97%, at least 98%, or at least 99% sequence identity thereto. The amino acid sequence of the heavy chain variable region is as shown in SEQ ID NO: 2; or has at least 70%, 75%, 80%, 85%, 90%, 95%, 96%, 97%, 98%, or 99% sequence identity thereto.

Specifically, the light chain variable region has a complementarity determining region comprising three sequences as shown in SEQ ID NO:3, SEQ ID NO:4, and SEQ ID NO:5, or a sequence having at least 70%, at least 75%, at least 80%, at least 85%, at least 90%, at least 95%, at least 96%, at least 97%, at least 98%, or at least 99% sequence identity to any one of SEQ ID NO:3, SEQ ID NO:4, and SEQ ID NO:5, and the heavy chain variable region has a complementarity determining region comprising three sequences as shown in SEQ ID NO:6, SEQ ID:7, and SEQ ID:8, or a sequence having at least 70%, at least 75%, at least 80%, at least 85%, at least 90%, at least 95%, at least 96%, at least 97%, at least 98%, or at least 99% sequence identity to any one of SEQ ID NO:6, SEQ ID:7, and SEQ ID:8.

In a second aspect, this application provides a hybridoma cell line designated as K64-1 and having an accession number of CCTCC NO: C2023365. The hybridoma cell line is used to prepare the monoclonal antibody directed to a capsular polysaccharide of *K. pneumoniae* as described in the previous aspect.

In a third aspect, this application provides a monoclonal antibody directed to a capsular polysaccharide of *K. pneumoniae,* wherein the *K. pneumoniae* is K64-type *K*. *pneumoniae,* the antibody has a sequence comprising a light chain variable region and a heavy chain variable region, the light chain variable region has an amino acid sequence as shown in SEQ ID NO:9, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:10, or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:11, SEQ ID NO:12, and SEQ ID NO:13, or a sequence having at least 70%, at least 75%, at least 80%, at least 85%, at least 90%, at least 95%, at least 96%, at least 97%, at least 98%, or at least 99% sequence identity to any one of SEQ ID NO:11, SEQ ID NO:12, and SEQ ID NO:13, and the heavy chain variable region has a complementarity determining region comprising three sequences as shown in SEQ ID:14, SEQ ID:15, and SEQ ID:16, or a sequence having at least 70%, at least 75%, at least 80%, at least 85%, at least 90%, at least 95%, at least 96%, at least 97%, at least 98%, or at least 99% sequence identity to any one of SEQ ID:14, SEQ ID:15, and SEQ ID:16.

In a fourth aspect, this application provides a hybridoma cell line designated as K64-23 and having an accession number of CCTCC NO: C2023366. The hybridoma cell line is used to prepare the monoclonal antibody directed to a capsular polysaccharide of *K. pneumoniae* as described in the third aspect.

In a fifth aspect, this application provides a monoclonal antibody directed to a capsular polysaccharide of *K. pneumoniae,* wherein the *K. pneumoniae* is K64-type *K*. *pneumoniae,* the antibody has a sequence comprising a light chain variable region and a heavy chain variable region, the light chain variable region has an amino acid sequence as shown in SEQ NO:17, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequences of SEQ ID NO:18 or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:19, SEQ ID NO:20, and SEQ ID NO:21, or a sequence having at least 70%, at least 75%, at least 80%, at least 85%, at least 90%, at least 95%, at least 96%, at least 97%, at least 98%, or at least 99% sequence identity to any one of SEQ ID NO:19, SEQ ID NO:20, and SEQ ID NO:21, and the heavy chain variable region has a complementarity determining region comprising three sequences as shown in SEQ ID:22, SEQ ID:23, and SEQ ID:24, or a sequence having at least 70%, at least 75%, at least 80%, at least 85%, at least 90%, at least 95%, at least 96%, at least 97%, at least 98%, or at least 99% sequence identity to any one of SEQ ID:22, SEQ ID:23, and SEQ ID:24.

In a sixth aspect, this application provides a hybridoma cell line designated as K64-24 and has an accession number of CCTCC NO: C2023367. The hybridoma cell line is used to prepare the monoclonal antibody directed to a capsular polysaccharide of *K. pneumoniae* as described in the fifth aspect.

In a seventh aspect, this application provides a monoclonal antibody directed to a capsular polysaccharide of *K. Pneumoniae,* wherein the *K. pneumoniae* is K64-type *K*. *pneumoniae,* the antibody has a sequence comprising a light chain variable region and a heavy chain variable region, the light chain variable region has an amino acid sequence as shown in SEQ ID NO:25, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:26, or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:27, SEQ ID NO:28, and SEQ ID NO:29, or a sequence having at least 70%, at least 75%, at least 80%, at least 85%, at least 90%, at least 95%, at least 96%, at least 97%, at least 98%, or at least 99% sequence identity to any one of SEQ ID NO:27, SEQ ID NO:28, and SEQ ID NO:29, and the heavy chain variable region has a complementarity determining region comprising three sequences as shown in SEQ ID:30, SEQ ID:31, and SEQ ID:32, or a sequence having at least 70%, at least 75%, at least 80%, at least 85%, at least 90%, at least 95%, at least 96%, at least 97%, at least 98%, or at least 99% sequence identity to any one of SEQ ID:30, SEQ ID:31, and SEQ ID:32.

In an eighth aspect, this application provides a hybridoma cell line designated as K64-3 and having an accession number CCTCC NO: C2024340. The hybridoma cell line is used to prepare the monoclonal antibody directed to a capsular polysaccharide of *K. pneumoniae* as described in the seventh aspect.

In a ninth aspect, this application provides a monoclonal antibody directed to a capsular polysaccharide of *K. pneumoniae,* wherein the *K. pneumoniae* is K64-type *K*. *pneumoniae,* the antibody has a sequence comprising a light chain variable region and a heavy chain variable region, the light chain variable region has an amino acid sequence as shown in SEQ ID NO:33, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:34, or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:35, SEQ ID NO:36, and SEQ ID NO:37, or a sequence having at least 70%, at least 75%, at least 80%, at least 85%, at least 90%, at least 95%, at least 96%, at least 97%, at least 98%, or at least 99% sequence identity to any one of SEQ ID NO:35, SEQ ID NO:36, and SEQ ID NO:37, and the heavy chain variable region has a complementarity determining region comprising three sequences as shown in SEQ ID:38, SEQ ID:39, and SEQ ID:40, or a sequence having at least 70%, at least 75%, at least 80%, at least 85%, at least 90%, at least 95%, at least 96%, at least 97%, at least 98%, or at least 99% sequence identity to any one of SEQ ID:38, SEQ ID:39, and SEQ ID:40.

In a tenth aspect, this application provides a hybridoma cell line designated as K64-5 and having an accession number of CCTCC NO: C2024341. The hybridoma cell line is used to prepare the monoclonal antibody directed to a capsular polysaccharide of *K. pneumoniae* as described in the ninth aspect.

In an eleventh aspect, this application provides a monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:41, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:42, or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:43, SEQ ID NO:44, and SEQ ID NO:45, or a sequence having at least 70%, at least 75%, at least 80%, at least 85%, at least 90%, at least 95%, at least 96%, at least 97%, at least 98%, or at least 99% sequence identity to any one of SEQ ID NO:43, SEQ ID NO:44, and SEQ ID NO:45, and the heavy chain variable region has a complementarity determining region comprising three sequences as shown in SEQ ID:46, SEQ ID:47, and SEQ ID:48, or a sequence having at least 70%, at least 75%, at least 80%, at least 85%, at least 90%, at least 95%, at least 96%, at least 97%, at least 98%, or at least 99% sequence identity to any one of SEQ ID:46, SEQ ID:47, and SEQ ID:48.

In a twelfth aspect, this application provides a hybridoma cell line designated as K64-8 and having an accession number of CCTCC NO: C2024342. The hybridoma cell line is used to prepare the monoclonal antibody directed to a capsular polysaccharide of *K. pneumoniae* as described in the eleventh aspect.

In a thirteenth aspect, this application provides a monoclonal antibody directed to a capsular polysaccharide of *K. pneumoniae,* wherein the *K. pneumoniae* is K64-type *K*. *pneumoniae,* the antibody has a sequence comprising a light chain variable region and a heavy chain variable region, the light chain variable region has an amino acid sequence as shown in SEQ ID NO:49, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:50, or a sequence having at least 70%, at least 75%, at least 80%, at least 85%, at least 90%, at least 95%, at least 96%, at least 97%, at least 98%, or at least 99% sequence identity thereto.

Specifically, the light chain variable region has a complementarity determining region comprising three sequences as shown in SEQ ID NO:51, SEQ ID NO:52, and SEQ ID NO:53, or a sequence having at least 70%, at least 75%, at least 80%, at least 85%, at least 90%, at least 95%, at least 96%, at least 97%, at least 98%, or at least 99% sequence identity to any one of SEQ ID NO:51, SEQ ID NO:52, and SEQ ID NO:53, and the heavy chain variable region has a complementarity determining region comprising three sequences as shown in SEQ ID:54, SEQ ID:55, and SEQ ID:56, or a sequence having at least 70%, at least 75%, at least 80%, at least 85%, at least 90%, at least 95%, at least 96%, at least 97%, at least 98%, or at least 99% sequence identity to any one of SEQ ID:54, SEQ ID:55, and SEQ ID:56.

In a fourteenth aspect, this application provides a hybridoma cell line designated as K64-27 and having an accession number of CCTCC NO: C2024343. The hybridoma cell line is used to prepare the monoclonal antibody directed to a capsular polysaccharide of *K. pneumoniae* as described in the fifteenth aspect.

In a fifteenth aspect, this application provides a pharmaceutical composition including the monoclonal antibody directed to a capsular polysaccharide of *K. pneumoniae* described in any one of the foregoing aspects. The antibody may bind to a target antigen of a capsular polysaccharide of *K. pneumoniae* and a target antigen other than a capsular polysaccharide of *K. pneumoniae.* The pharmaceutical composition further includes any one or a combination of more than one of a pharmaceutically acceptable carrier, an excipient, or a diluent.

In a sixteenth aspect, this application provides a kit for detecting K64-type *K*. *pneumoniae.* The kit includes the monoclonal antibody directed to a capsular polysaccharide of *K. pneumoniae* described in any one of the foregoing aspects. The kit further includes any one or a combination of more than one of a positive control article, a negative control article, an antibody dilution, a chromogen solution, a stop solution, a blocking solution, or a wash solution.

In a seventeenth aspect, this application provides an application of the monoclonal antibody directed to a capsular polysaccharide of *K. pneumoniae* of any one of the foregoing aspects in preparing a therapeutic drug and/or a detection product for infectious diseases caused by K64-type *K. pneumoniae.* The disease includes pneumonia, urinary tract infections, sepsis/bacteremia/sepsis, neonatal sepsis I bacteremia/sepsis, diarrhea, soft tissue infections, post-organ transplantation infections, surgical infections, wound infections, lung infections, pyogenic liver abscess, lung abscess, cellulitis, necrotizing fasciitis, myositis, endophthalmitis, peritonitis, meningitis, necrotizing meningitis, or spondyloarthropathy.

The capsular polysaccharide is an important toxicological factor of *K. pneumoniae.* In this application, the capsular polysaccharide is coupled with a carrier protein to improve immunogenicity. The monoclonal antibody directed to a capsular polysaccharide of K64-type *K. pneumoniae* prepared through a hybridoma technology has the characteristics of good specificity, high affinity, and high binding ability to the capsular polysaccharide of *K. pneumoniae.*

Beneficial effects of this application are as follows.

The monoclonal antibody directed to a capsular polysaccharide of *K. pneumoniae* provided in this application can specifically bind to the capsular polysaccharide of K64-type *K. pneumoniae,* facilitating serotyping of *K. pneumoniae* in clinical practice. Both in-vivo and in-vitro animal tests demonstrate that the antibody has excellent effects in preventing and treating infections caused by K64-type *K. pneumoniae,* which provides a way to solve problems of *K. pneumoniae* infections and multi-drug resistance, has significant significance in the prevention, diagnosis, and treatment of *K. pneumoniae* infections, and has an important value for the development of new-generation drugs against *K. pneumoniae.*

### BRIEF DESCRIPTION OF THE DRAWINGS

FIG. 1 is a diagram of a detection result of a titer level of a monoclonal antibody directed to a capsular polysaccharide of *K. pneumoniae* provided in this application, as detected through indirect ELISA in Example 2;
FIG. 2 is a graph of binding results of a K64 monoclonal antibody and a negative control with K64-type *K. pneumoniae,* Y8, as detected through flow cytometry in Example 4;
FIG. 3 shows in-vitro bactericidal curves of monoclonal antibodies K64-1, K64-23, and K64-24 against a hypervirulent *K. pneumoniae* strain (No. Y8) in Example 5; and
FIG. 4 shows the survival curve from the prophylactic test of the K64-1 antibody in Example 6.

### DETAILED DESCRIPTION OF THE DISCLOSURE

Technical solutions of this application are clearly described below. Obviously, the described embodiments are part of, rather than all, the embodiments of this application. All other embodiments obtained by a person of ordinary skill in the art based on the embodiments of this application without inventive effort fall within the scope of protection of this application.

The following is strain preservation information:
The hybridoma cell line K64-1 (CCTCC NO: C2023365), hybridoma cell line K64-23 (CCTCC NO: C2023366), and hybridoma cell line K64-24 (CCTCC NO: C2023367) provided in this application were deposited at the China Center for Type Culture Collection on November 28, 2023.
The hybridoma cell line K64-3 (CCTCC NO: C2024340), hybridoma cell line K64-5 (CCTCC NO: C2024341), hybridoma cell line K64-8 (CCTCC NO: C2024342), and hybridoma cell line K64-27 (CCTCC NO: C2024343) provided in this application were all deposited in the China Center for Type Culture Collection on September 25, 2024.

Accession Address: Wuhan University, Wuhan City, Hubei Province.

Accession Name and Accession Number:
Hybridoma cell line K64-1: Hybridoma cell line K64-1, CCTCC NO: C2023365.
Hybridoma cell line K64-23: Hybridoma cell line K64-23, CCTCC NO: C2023366.
Hybridoma cell line K64-24: Hybridoma cell line K64-24, CCTCC NO: C2023367.
Hybridoma cell line K64-3: Hybridoma cell line K64-3, CCTCC NO: C2024340.
Hybridoma cell line K64-5: Hybridoma cell line K64-5, CCTCC NO: C2024341.
Hybridoma cell line K64-8: Hybridoma cell line K64-8, CCTCC NO: C2024342.
Hybridoma cell line K64-27: Hybridoma cell line K64-27, CCTCC NO: C2024343.

In Example 4 and Example 5 of this application, a sequence of a light chain variable region (VL) of a used negative control antibody (as shown in SEQ ID NO: 57) is:

MESHSQVFIFLLFWIPVSRGDILLTQSPAILSVSPGERVSFSCRASQSIGRSI
HWYQQRTNGSPRLLIKYASESIYGIPSRFSGSGTDFTLSINSVESEDIAAYYCQQSYN

WPRTFGGGTKLEIK

A sequence of a heavy chain variable region (VH) of a used negative control antibody (as shown in SEQ ID NO: 58) is:

MNFGLSWVFLVALLNGVQCQVQLVETGGGLVRPGNSLLSCITSGFTFSNY
RLHWLRQPPGKGLEWLAVIAVKSDNFGAIYADSVKGRFTISRDDSRSSVYLQMNR
LREEDTATYYCVRAGVSFFDYWGQGTSLTVSS

### Example 1: Preparation of Monoclonal Antibody Directed to Capsular Polysaccharide of K64-type K. pneumoniae

### 1) Establishment of Cell Line for Monoclonal Antibody Directed to Capsular Polysaccharide of K64-type K. pneumoniae

A capsular polysaccharide extracted after fermentation of K64-type *K*. *pneumoniae* (GDMCC No: 62132) and a carrier protein (a non-toxic variant CRM197 of diphtheria toxoid) were chemically coupled to obtain a K64-type capsular polysaccharide protein conjugate through a conventional polysaccharide activation method, such as a cyanogen bromide method (referring to US6375846B1), 1-cyano-4-dimethylaminopyridine tetrafluoroborate method (CDAP) (EP0720485), and a periodic acid oxidation method (US4711779). The K64-type capsular polysaccharide protein conjugate was added to a Freund's adjuvant. After a plurality of immunizations of SPF BALB/c mice, splenocytes thereof were harvested when the immunizing potency exceeded 1: 300,000. Using the hybridoma technology which involved fusion with PEG1500, screening of positive cell supernatants by indirect ELISA, and a plurality of rounds of subcloning, 3 monoclonal cell lines were obtained, which hybridoma cell line K64-1 (CCTCC NO: C2023365), hybridoma cell line K64-23 (CCTCC NO: C2023366), hybridoma cell line K64-24 (CCTCC NO: C2023367), hybridoma cell line K64-3 (CCTCC NO: C2024340), hybridoma cell line K64-5 (CCTCC NO: C2024341), hybridoma cell line K64-8 (CCTCC NO: C2024342), and hybridoma cell line K64-27 (CCTCC NO: C2024343). Sequencing verified that they were pure.

After sequencing, the sequence information of 7 monoclonal antibodies directed to a capsular polysaccharide of *K. pneumoniae* is as follows (sequentially SEQ NO: 1-SEQ NO: 56):
a sequence (starting from FR-L1) of a light chain variable region (VL) of K64-1: 
QIVLTQSPAIMSASPGEKVTMTCSASSSISYMHWYQQKPGTSPKRWIYDT
SKLASGVPARFSGSGTSYSLTISSMEAEDAATYYCHQRSSYPWTFGGGTKLEIK
a sequence (starting from FR-H1) of a heavy chain variable region (VH) of K64-1: 
EVKLVESGGGLVKPGGSLKLSCAGSGFTFSSYGMSWVRQTPDKRLEWVA
TISGGGSYTYYSDSVKGRFTISRDNAKNNLYLQMSSLRSEDTALYYYCARNYGYD
GHYYAMDYWGQGTSVTVSS
a sequence of a complementarity determining region 1 (LCDR1) of a light chain of K64-1:
   SASSSISYMH
a sequence of a complementarity determining region 2 (LCDR2) of a light chain of K64-1:
   DTSKLAS
a sequence of a complementarity determining region 3 (LCDR3) of a light chain of K64-1:
   HQRSSYPWT
a sequence of a complementarity determining region 1 (HCDR1) of a heavy chain of K64-1:
   SYGMS
a sequence of a complementarity determining region 2 (HCDR2) of a heavy chain of K64-1:
   TISGGGSYTYYSDSVKG
a sequence of a complementarity determining region 3 (HCDR3) of a heavy chain of K64-1:
   NYGYDGHYYAMDY
a sequence (starting from FR-L1) of a light chain variable region (VL) of K64-23: 
QIVLTQSPAIMSASPGEKVTMTCSATSIVNYMHWYQQKSGTSPKRWIYDT
SKLASGVPARFSGSGTSYSLTISSMEAEDAATYYCQQWSSNPRT
a sequence (starting from FR-H1) of a heavy chain variable region (VH) of K64-23: QVQLQQPWSELVRPGASVKLPCKASGYTFTSYWMNWVKQRPEQGLEWI 
GRIDPYDGGTHYSQNFMDRAILTVDRSSNTAYMQLNSLTSEDSAVYYCARGYANG

AFVYWGQGTLVTVSA
a sequence of a complementarity determining region 1 (LCDR1) of a light chain of K64-23:
   SATSIVNYMH
a sequence of a complementarity determining region 2 (LCDR2) of a light chain of K64-23:
   DTSKLAS
a sequence of a complementarity determining region 3 (LCDR3) of a light chain of K64-23:
   QQWSSNPRT
a sequence of a complementarity determining region 1 (HCDR1) of a heavy chain of K64-23:
   SYWMN
a sequence of a complementarity determining region 2 (HCDR2) of a heavy chain of K64-23:
   RIDPYDGGTHYSQNFMD
a sequence of a complementarity determining region 3 (HCDR3) of a heavy chain of K64-23:
   GYANGAFVY
a sequence (starting from FR-L1) of a light chain variable region (VL) of K64-24: 
QIVLTQSPAIMSASPGEKVTISCSASSSVSYMYWYQQRPGSSPKPWIYRTS
NLASGVPARFSGSGTSYSLTISSMEAEDAATYYCQQYHSYPLTFGAGTKLELK
a sequence (starting from FR-H1) of a heavy chain variable region (VH) of K64-24: 
EVKLEESGGGLVQPGGSMKLSCAASGFTFSDAYMDWVRQSPEKGLEWV
AEIRSKANNHATLYAESVKGRFTISRDDSKSSVYLQMNSLRAEDTGIYYCTPYYYG
SSQFAYWGQGTLVTVSA
a sequence of a complementarity determining region 1 (LCDR1) of a light chain of K64-24:
   SASSSVSYMY
a sequence of a complementarity determining region 2 (LCDR2) of a light chain of K64-24:
   RTSNLAS
a sequence of a complementarity determining region 3 (LCDR3) of a light chain of K64-24:
   QQYHSYPLT
a sequence of a complementarity determining region 1 (HCDR1) of a heavy chain of K64-24:
   DAYMD
a sequence of a complementarity determining region 2 (HCDR2) of a heavy chain of K64-24:
   EIRSKANNHATLYAESVKG
a sequence of a complementarity determining region 3 (HCDR3) of a heavy chain of K64-24:
   YYYGSSQFAY
a sequence (starting from FR-L1) of a light chain variable region (VL) of K64-3: 
QIVLTQSPAIMSASPGEKVTMTCSASSSISYMHWYQQKPGTSPKRWIYDT
SKLASGVPARFSGSGTSYSLTISSMEAEDAATYYCHQRSSYPWTFGGGTKLEIK
a sequence (starting from FR-H1) of a heavy chain variable region (VH) of K64-3: 
EVKLVESGGGLVKPGGSLTLSCAASGSIFSTYGMSWVRQTPEKRLEWVAT
ISGGGRYTYYPDSVKGRFTISRDNAKNNLYLQMSSLRSEDTALYYCARNYGYDGH
YYPMDYWGQGTSVTVSS
a sequence of a complementarity determining region 1 (LCDR1) of a light chain of K64-3:
   SASSSISYMH
a sequence of a complementarity determining region 2 (LCDR2) of a light chain of K64-3:
   DTSKLAS
a sequence of a complementarity determining region 3 (LCDR3) of a light chain of K64-3:
   HQRSSYPWT
a sequence of a complementarity determining region 1 (HCDR1) of a heavy chain of K64-3:
   TYGMS
a sequence of a complementarity determining region 2 (HCDR2) of a heavy chain of K64-3:
   TISGGGRYTYYPDSVKG
a sequence of a complementarity determining region 3 (HCDR3) of a heavy chain of K64-3:
   NYGYDGHYYPMDY
a sequence (starting from FR-L1) of a light chain variable region (VL) of K64-5: 
QIVLTQSPAIMSASPGEKVTMTCSASSSISYMHWYQQKPGTSPKRWIYDT
SKLASGVPVRFSGSGTSYSLTISSMEAEDAATYYCHQRSSYPWTFGGGTKLEIK
a sequence (starting from FR-H1) of a heavy chain variable region (VH) of K64-5: 
EVKLVESGGGLVKPGGSLELSCAASGFTFSTYGMSWVRQTPEKRLEWVA

TISGGGSYTYYVDSVKGRFTISRDNAKNNLYLQMSSLRSEDTALYYCARNYGYDG

HYYSMDYWGQGTSVTVSS
a sequence of a complementarity determining region 1 (LCDR1) of a light chain of K64-5:
   SASSSISYMH
a sequence of a complementarity determining region 2 (LCDR2) of a light chain of K64-5:
   DTSKLAS
a sequence of a complementarity determining region 3 (LCDR3) of a light chain of K64-5:
   HQRSSYPWT
a sequence of a complementarity determining region 1 (HCDR1) of a heavy chain of K64-5:
   TYGMS
a sequence of a complementarity determining region 2 (HCDR2) of a heavy chain of K64-5:
   TISGGGSYTYYVDSVKG
a sequence of a complementarity determining region 3 (HCDR3) of a heavy chain of K64-5:
   NYGYDGHYYSMDY
a sequence (starting from FR-L1) of a light chain variable region (VL) of K64-8: 
QIVLTQSPAIMSASPGEKVTMTCSASSSISYMHWYQQKPGTSPKRWIYDT
SKLASGVPARFSGSGTSYSLTISSMEAEDAATYYCHQRSNYPWTFGGGTKLEIK
a sequence (starting from FR-H1) of a heavy chain variable region (VH) of K64-8: 
EVKLVESGGDLVKPGGSLKLSCAASGFTFSSYGMSWVRQTPEKRLEWVA
TISGGGYYTYYPDSVKGRFTISRDNAKNNLYLQMSSLRSEDTALYYCGRNYGYDG
HYYAMDYWGQGTSVTVSS
a sequence of a complementarity determining region 1 (LCDR1) of a light chain of K64-8:
   SASSSISYMH
a sequence of a complementarity determining region 2 (LCDR2) of a light chain of K64-8:
   DTSKLAS
a sequence of a complementarity determining region 3 (LCDR3) of a light chain of K64-8:
   HQRSNYPWT
a sequence of a complementarity determining region 1 (HCDR1) of a heavy chain of K64-8:
   SYGMS
a sequence of a complementarity determining region 2 (HCDR2) of a heavy chain of K64-8:
   TISGGGYYTYYPDSVKG
a sequence of a complementarity determining region 3 (HCDR3) of a heavy chain of K64-8:
   NYGYDGHYYAMDY
a sequence (starting from FR-L1) of a light chain variable region (VL) of K64-27: 
QIVLTQSPAIMSASPGEKVTISCSASSSVSYMYWYQQKPGSSPKPWIYRTS
NLASGVPARFSGSGTSYSLTISSMEAEDAATYYCQQYHSYPLTFGAGTKLELK
a sequence (starting from FR-H1) of a heavy chain variable region (VH) of K64-27: 
EVKLEESGGGLVQPGGSMKLSCAASGFTFSDAYMDWVRQSPEKGLEWV
AEIRSKANNHATYYAESVKGRFTISRDDSKSSVYLQMNSLRAEDTGIYYCTPYYY
GSSQFAYWGQGTLVTVSA
a sequence of a complementarity determining region 1 (LCDR1) of a light chain of K64-27:
   SASSSVSYMY
a sequence of a complementarity determining region 2 (LCDR2) of a light chain of K64-27:
   RTSNLAS
a sequence of a complementarity determining region 3 (LCDR3) of a light chain of K64-27:
   QQYHSYPLT
a sequence of a complementarity determining region 1 (HCDR1) of a heavy chain of K64-27:
   DAYMD
a sequence of a complementarity determining region 2 (HCDR2) of a heavy chain of K64-27:
   EIRSKANNHATYYAESVKG
a sequence of a complementarity determining region 3 (HCDR3) of a heavy chain of K64-27:
   YYYGSSQFAY.

As used in this application, the term "CDR" or "a complementarity determining region" refers to non-contiguous antigen-binding sites found in variable regions of heavy and light chain polypeptides. In this application, the amino acid sequences of the CDRs listed above are shown based on the Kabat definition rule (the claims of the present invention also show the sequences based on the Kabat definition rule). However, those skilled in the art know that a CDR of an antibody may be defined in a plurality of ways in the art. In the documents, Kabat et al., J. Biol. Chem. 252: 6609-6616 (1977); Kabat et al, U.S. Dept. of Health and Human Services, "Sequences of proteins of immunological interest" (1991); Chothia et al, J. Mol. Biol. 196: 901-917 (1987); Al-Lazikani B. et al., J. Mal. Biol, 273: 927-948 (1997); MacCallum et al, J. Mol. Biol. 262: 732-745 (1996); Abhinandan and Martin, Mal Immunol, 45: 3832-3839 (2008); Lefranc M.P. et al., Dev. Comp. Jmmunol, 27: 55-77 (2003); and Honegger and Pltickthun, J. Mal Biol., 309: 657-670 (2001), these special regions are described, where these definitions include coincidence or a subset of amino acid residues when compared to each other. However, a CDR of an antibody or a grafted antibody or variant thereof, indicated by any definition manner, is included within the scope of the terms defined and used in this application. An algorithm and a binding interface for CDR prediction are known in the art, such as, described in Abhinandan and Martin, Mal. Immunol, 45: 3832-3839 (2008); Ehrenmann F. et al, Nucleic Acids Res., 38: D301-D307 (2010); and Adolf-Bryfogle J. et al., Nucleic Acids Res., 43: D432-D438 (2015). The content of the reference documents cited in this paragraph is incorporated herein by reference in its entirety for use in this application and in one or more claims that may be included in this application.

As used herein, "monoclonal antibody" refers to a homologous antibody population that specifically recognizes and binds to a single antigenic determinant or epitope, including both complete and full-length monoclonal antibodies and antibody fragments (such as Fab, Fab ', F (ab') 2, Fv), single-chain (scFv) mutants, fusion proteins comprising antibody portions, and any other modified immunoglobulin molecules, including antigen recognition sites. Furthermore, "monoclonal antibodies" include such antibodies prepared in any manner (including, but not limited to, hybridoma, a phage selection, recombinant expression, and transgenic animals). In humans, this category includes IgG1, IgG2, IgG3, and IgG4. In mice, this category includes IgG1, IgG2a, IgG2b, and IgG3. As is known in the art, the antigen-binding function of an antibody may be performed by a fragment of a full-length antibody. The monoclonal antibody or antigen-binding fragment described in the claims of the present invention includes an Fc fragment of the foregoing categories of antibodies.

### 2) Preparation and Purification of Monoclonal Antibody Directed to Capsular Polysaccharide of K64-type K. pneumoniae from Ascites

The obtained hybridoma cells were expanded in culture. BALB/c mice, which had been primed with paraffin oil 7 days in advance, were intraperitoneally injected with 0.5 mL of the cell suspension at a density of 1 × 10⁶ cells/mL. After 7-10 days, the mice were observed to have significant abdominal distension, and the ascites thereof were harvested for purification.

After the ascites were diluted with PBS, ammonium sulfate with a final concentration of 50% was added for crude purification by salt precipitation. A monoclonal antibody with a purity greater than 95% was obtained through ProteinA affinity column chromatography and ion exchange chromatography. After sterile filtration through a 0.22-µm filter membrane, the antibody was stored in PBS at a pH of 7.0-7.4.

### Example 2: Detection of Titer Level of Monoclonal Antibody Directed to Capsular Polysaccharide of K64-type K. pneumoniae

After cell fusion, screening, and several rounds of subcloning, 7 monoclonal antibody cell lines were obtained. The results of the detection of binding of cell culture supernatants thereof to a K64-type capsular polysaccharide through ELISA are shown in Table 1 below.

**Table 1**

| Cell Line Number | OD Value |
|---|---|
| K64-1 | 3.816 |
| K64-23 | 1.315 |
| K64-24 | 2.011 |
| K64-3 | 3.227 |
| K64-5 | 0.707 |
| K64-8 | 3.362 |
| K64-27 | 1.043 |

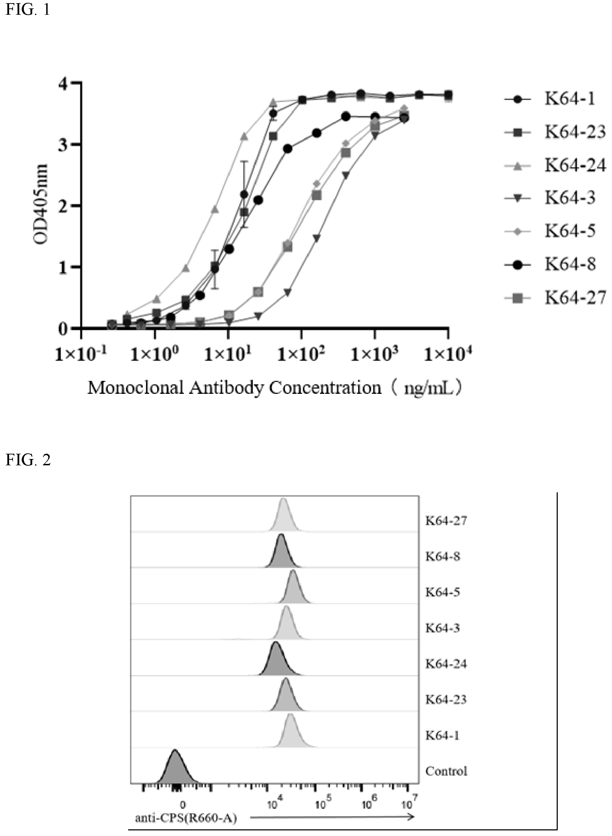

As shown in FIG. 1, the titer level of the monoclonal antibody directed to a capsular polysaccharide of *K. pneumoniae* provided in this application is detected through an indirect ELISA method. It can be seen that supernatants from all 7 cell lines exhibit strong binding to the K64-type capsular polysaccharide.

### Example 3: Detection of Binding Specificity of Monoclonal Antibody Directed to Capsular Polysaccharide of K64-type K. pneumoniae through ELISA

The specificity of the monoclonal antibody directed to a capsular polysaccharide of *K. pneumoniae* provided in this application was evaluated through indirect ELISA to analyze the reactivity thereof with K47-type, K64-type, K19-type, K1-type, K2-type, K38-type, and K57-type capsular polysaccharides. Enzyme-labeled plates were coated with *Klebsiella* capsular polysaccharides of different serotypes at 37°C for 3 hours and blocked with 1% BSA for 1 hour. Then, an appropriate amount of a monoclonal antibody directed to a capsular polysaccharide of K64-type *K. pneumoniae* was added to react with the coated polysaccharide, with 50 µL per well, and incubated overnight. After plate washing, an AP-conjugated goat anti-mouse secondary antibody was diluted at 1: 30,000 and added to an enzyme-labeled plate, with 100 µL per well. After incubation for 2 hours, the plates were washed. Then, a 1 mg/mL PNPP-Na chromogenic substrate was added, with 100 µL per well. After incubation for 2 hours, 50µL/well of 3M NaOH was added to stop the reaction. An OD405 value was measured.

The results of binding of the 7 monoclonal antibodies directed to a capsular polysaccharide of K64-type *K. pneumoniae* provided in this application to *Klebsiella* capsular polysaccharides of different serotypes are shown in Table 2 below:

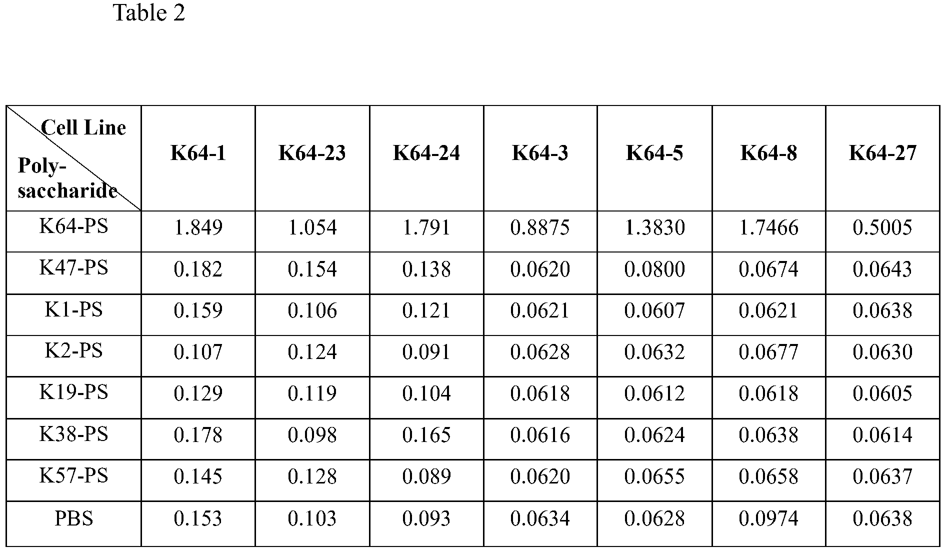

As can be seen from Table 2 above, the 7 monoclonal antibodies directed to a capsular polysaccharide of K64-type *K. pneumoniae* provided in this application have specificity for the K64-type capsular polysaccharide, but enable no cross-reactions with *Klebsiella* capsular polysaccharides having other serotypes.

### Example 4: Detection of Binding of Monoclonal Antibody Directed to Capsular Polysaccharide of K64-type K. pneumoniae to K64-type K. pneumoniae through Flow Cytometric

Hypervirulent K64-type *K. pneumoniae,* Y8 (Li, J.; Sheng, Y; Ma, R.; Xu, M.; Liu, F.; Qin, R.; Zhu, M.; Zhu, X.; He, P. Identification of a Depolymerase Specific for K64-type K. pneumoniae: Potential Applications in Capsular Serotyping and Treatment. Antibiotics 2021, 10, 144.) is inoculated into 5 ml LB medium for culture at 37 °C and 250 rpm until an OD value of 0.7. 0.2 mL of a bacterial solution is taken and centrifuged at 8000 g for 10 min. A precipitate is collected, washed twice with PBS, and resuspended in 0.2 mL PBS. The bacteria are immobilized with 4% paraformaldehyde for 10 min, blocked with 2% BSA for 1 hour after washing, and incubated overnight with a monoclonal antibody directed to the K64-type capsular polysaccharide. The bacteria are washed, added with an anti-mouse fluorescent antibody (Brand-CST, Item No.-# 4410S) after washing, incubated in the dark for 1 hour, resuspended in PBS after being washed with PBS, and analyzed through flow cytometry.

After the hypervirulent *K. pneumoniae* strain Y8 is cultured, its binding ability to K64-type monoclonal antibodies is analyzed through flow cytometry. Test results are shown in FIG. 2. Seven K64-type monoclonal antibodies are enabled to bind to the hypervirulence K64-type *K. pneumoniae* strain Y8, and detection is made. Results show that all 7 K64-type monoclonal antibodies can bind to Y8, while the negative control does not bind to Y8, indicating that these 7 K64-type monoclonal antibodies directed to a capsular polysaccharide may be applied to the diagnosis and serotyping of clinical strains.

### Example 5: Bactericidal Test of Monoclonal Antibody Directed to Capsular Polysaccharide of K64-type K. pneumoniae

This example shows the opsonophagocytic killing assay for the monoclonal antibody directed to a capsular polysaccharide of K64-type *K. pneumoniae* provided in this application. Specifically, a hypervirulent K64-type *K. pneumoniae* strain Y8 is diluted to 10⁵ CFU/ml and added to a 96-well cell culture plate at 10 µL/well. After gradient dilution of a monoclonal antibody directed to the K64-type capsular polysaccharide, the antibody is added to the above cell culture plate at 20 µL/well. The bacteria and the antibodies are incubated at 700 rpm/min for 30 min. After being washed with an HBSS buffer, HL-60 cells differentiated through DMF are adjusted to a concentration of 1×10⁷ cells/ml, and this cell solution with 1×10⁷ cells/ml is mixed with a diluted complement at a volume ratio of 1: 4. The mixture is added to a 96-well plate with 50 µL/well. The 96-well cell culture plate is placed on a mixer inside a 37°C incubator with 5% CO₂ and shaken for cultivation for 45 min. After the assay ends, samples are loaded into blood agar plates and incubated overnight in a CO₂ incubator. The bactericidal rate for each diluted antibody is calculated.

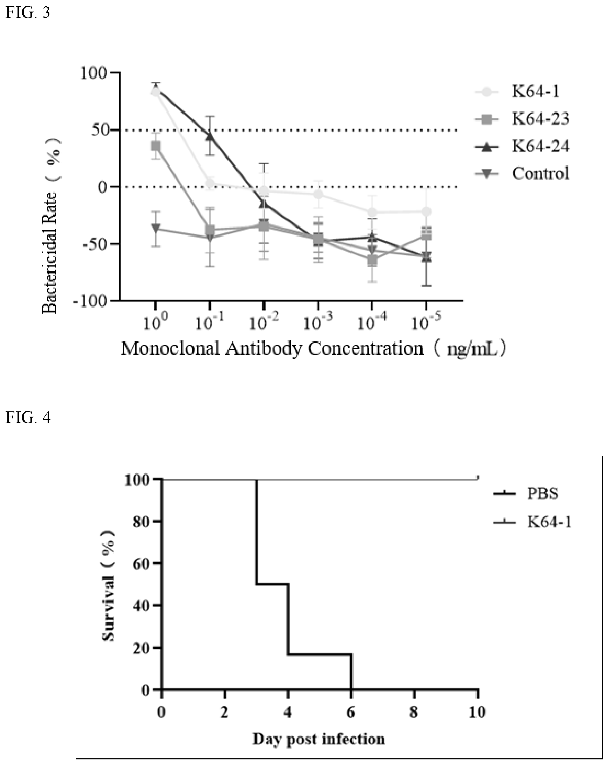

As shown in FIG. 3, the K64-type monoclonal antibodies K64-1, K64-23, and K64-24 against a capsular polysaccharide exhibited potent in vitro bactericidal activity against the hypervirulent strain Y8. The peak killing rates reach 83.3% and 86.7%, respectively, showing a significant difference compared to the negative control antibody.

### Example 6: Prophylactic Efficacy of Monoclonal Antibody Directed to Capsular Polysaccharide of K64-type K. pneumoniae

This example demonstrates the protective test of the monoclonal antibody directed to a capsular polysaccharide of *K. pneumoniae* provided in this application, specifically as follows. The monoclonal antibody K64-1 against a capsular polysaccharide of K64-type *K. pneumoniae* is administered via intraperitoneal injection to 6 mice in each group with 0.025 mg per mouse. PBS is used as a negative control. After 4 hours, Balb/c mice (n=6) are infected with a hypervirulent *K. pneumoniae* strain Y8 at a bacterial amount of 7.38×10⁶ CFU. After the infection, mouse survival is monitored for 10 days post-infection, and a survival curve is plotted.

As shown in FIG. 4, it may be seen that after 4 hours of the administration of the monoclonal antibody K64-1 against the K64-type capsular polysaccharide and the control PBS, a lethal challenge with 7.38 × 10⁶ CFU of K64-type *K. Pneumoniae* (Strain Y8) was conducted. The control group exhibited a 0% survival rate, whereas the monoclonal antibody group shows a 100% survival rate, enabling a statistical difference (p<0.05).

Based on the above, the foregoing embodiments are merely preferred embodiments of this application and are not intended to limit the scope of protection of this application. Any modification, equivalent replacement, improvement, or the like made without departing from the spirit and principle of this application shall fall within the protection scope of this application.

## Claims

1. A monoclonal antibody directed to a capsular polysaccharide of *Klebsiella pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO: 1, or a sequence with at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:2 or a sequence having at least 70%, at least 75%, at least 80%, at least 85%, at least 90%, at least 95%, at least 96%, at least 97%, at least 98%, or at least 99% sequence identity thereto.

2. The monoclonal antibody directed to a capsular polysaccharide of *K. pneumoniae* according to claim 1, wherein the light chain variable region has a complementarity determining region comprising three sequences as shown in SEQ ID NO:3, SEQ ID NO:4, and SEQ ID NO:5, or a sequence having at least 70%, at least 75%, at least 80%, at least 85%, at least 90%, at least 95%, at least 96%, at least 97%, at least 98%, or at least 99% sequence identity to any one of SEQ ID NO:3, SEQ ID NO:4, and SEQ ID NO:5, and the heavy chain variable region has a complementarity determining region comprising three sequences as shown in SEQ ID NO:6, SEQ ID:7, and SEQ ID:8, or a sequence having at least 70%, at least 75%, at least 80%, at least 85%, at least 90%, at least 95%, at least 96%, at least 97%, at least 98%, or at least 99% sequence identity to any one of SEQ ID NO:6, SEQ ID:7, and SEQ ID:8.

3. A hybridoma cell line designated as K64-1 and having an accession number of CCTCC NO: C2023365, wherein the hybridoma cell line is used to prepare the monoclonal antibody directed to a capsular polysaccharide of *K. pneumoniae* according to claim 1.

4. A monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:9, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO: 10, or a sequence having at least 70%, at least 75%, at least 80%, at least 85%, at least 90%, at least 95%, at least 96%, at least 97%, at least 98%, or at least 99% sequence identity thereto.

5. The monoclonal antibody directed to a capsular polysaccharide of *K. pneumoniae* according to claim 4, wherein the light chain variable region has a complementarity determining region comprising three sequences as shown in SEQ ID NO:11, SEQ ID NO:12, and SEQ ID NO:13, or a sequence having at least 70%, at least 75%, at least 80%, at least 85%, at least 90%, at least 95%, at least 96%, at least 97%, at least 98%, or at least 99% sequence identity to any one of SEQ ID NO:11, SEQ ID NO:12, and SEQ ID NO:13, and the heavy chain variable region has a complementarity determining region comprising three sequences as shown in SEQ ID:14, SEQ ID:15, and SEQ ID:16, or a sequence having at least 70%, at least 75%, at least 80%, at least 85%, at least 90%, at least 95%, at least 96%, at least 97%, at least 98%, or at least 99% sequence identity to any one of SEQ ID:14, SEQ ID:15, and SEQ ID:16.

6. A hybridoma cell line designated as K64-23 and having an accession number of CCTCC NO: C2023366, wherein the hybridoma cell line is used to prepare the monoclonal antibody directed to a capsular polysaccharide of *K. pneumoniae* according to claim 4.

7. A monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:17, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence of SEQ ID NO: 18 or a sequence having at least 70%, at least 75%, at least 80%, at least 85%, at least 90%, at least 95%, at least 96%, at least 97%, at least 98%, or at least 99% sequence identity thereto.

8. The monoclonal antibody directed to a capsular polysaccharide of *K. pneumoniae* according to claim 7, wherein the light chain variable region has a complementarity determining region comprising three sequences as shown in SEQ ID NO:19, SEQ ID NO:20, and SEQ ID NO:21, or a sequence having at least 70%, at least 75%, at least 80%, at least 85%, at least 90%, at least 95%, at least 96%, at least 97%, at least 98%, or at least 99% sequence identity to any one of SEQ ID NO:19, SEQ ID NO:20, and SEQ ID NO:21, and the heavy chain variable region has a complementarity determining region comprising three sequences as shown in SEQ ID:22, SEQ ID:23, and SEQ ID:24, or a sequence having at least 70%, at least 75%, at least 80%, at least 85%, at least 90%, at least 95%, at least 96%, at least 97%, at least 98%, or at least 99% sequence identity to any one of SEQ ID:22, SEQ ID:23, and SEQ ID:24.

9. A hybridoma cell line designated as K64-24 and having an accession number of CCTCC NO: C2023367, wherein the hybridoma cell line is used to prepare the monoclonal antibody directed to a capsular polysaccharide of *K. pneumoniae* according to claim 7.

10. A monoclonal antibody directed to a capsular polysaccharide of *K. Pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:25, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:26, or a sequence having at least 70%, at least 75%, at least 80%, at least 85%, at least 90%, at least 95%, at least 96%, at least 97%, at least 98%, or at least 99% sequence identity thereto.

11. The monoclonal antibody directed to a capsular polysaccharide of *K. pneumoniae* according to claim 10, wherein the light chain variable region has a complementarity determining region comprising three sequences as shown in SEQ ID NO:27, SEQ ID NO:28, and SEQ ID NO:29, or a sequence having at least 70%, at least 75%, at least 80%, at least 85%, at least 90%, at least 95%, at least 96%, at least 97%, at least 98%, or at least 99% sequence identity to any one of SEQ ID NO:27, SEQ ID NO:28, and SEQ ID NO:29, and the heavy chain variable region has a complementarity determining region comprising three sequences as shown in SEQ ID:30, SEQ ID:31, and SEQ ID:32, or a sequence having at least 70%, at least 75%, at least 80%, at least 85%, at least 90%, at least 95%, at least 96%, at least 97%, at least 98%, or at least 99% sequence identity to any one of SEQ ID:30, SEQ ID:31, and SEQ ID:32.

12. A hybridoma cell line designated as K64-3 and having an accession number of CCTCC NO: C2024340, wherein the hybridoma cell line is used to prepare the monoclonal antibody directed to a capsular polysaccharide of *K. pneumoniae* according to claim 10.

13. A monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:33, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:34, or a sequence having at least 70%, at least 75%, at least 80%, at least 85%, at least 90%, at least 95%, at least 96%, at least 97%, at least 98%, or at least 99% sequence identity thereto.

14. The monoclonal antibody directed to a capsular polysaccharide of *K. pneumoniae* according to claim 13, wherein the light chain variable region has a complementarity determining region comprising three sequences as shown in SEQ ID NO:35, SEQ ID NO:36, and SEQ ID NO:37, or a sequence having at least 70%, at least 75%, at least 80%, at least 85%, at least 90%, at least 95%, at least 96%, at least 97%, at least 98%, or at least 99% sequence identity to any one of SEQ ID NO:35, SEQ ID NO:36, and SEQ ID NO:37, and the heavy chain variable region has a complementarity determining region comprising three sequences as shown in SEQ ID:38, SEQ ID:39, and SEQ ID:40, or a sequence having at least 70%, at least 75%, at least 80%, at least 85%, at least 90%, at least 95%, at least 96%, at least 97%, at least 98%, or at least 99% sequence identity to any one of SEQ ID:38, SEQ ID:39, and SEQ ID:40.

15. A hybridoma cell line designated as K64-5 and having an accession number of CCTCC NO: C2024341, wherein the hybridoma cell line is used to prepare the monoclonal antibody directed to a capsular polysaccharide of *K. pneumoniae* according to claim 13.

16. A monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:41, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:42, or a sequence having at least 70%, at least 75%, at least 80%, at least 85%, at least 90%, at least 95%, at least 96%, at least 97%, at least 98%, or at least 99% sequence identity thereto.

17. The monoclonal antibody directed to a capsular polysaccharide of *K. pneumoniae* according to claim 16, wherein the light chain variable region has a complementarity determining region comprising three sequences as shown in SEQ ID NO:43, SEQ ID NO:44, and SEQ ID NO:45, or a sequence having at least 70%, at least 75%, at least 80%, at least 85%, at least 90%, at least 95%, at least 96%, at least 97%, at least 98%, or at least 99% sequence identity to any one of SEQ ID NO:43, SEQ ID NO:44, and SEQ ID NO:45, and the heavy chain variable region has a complementarity determining region comprising three sequences as shown in SEQ ID:46, SEQ ID:47, and SEQ ID:48, or a sequence having at least 70%, at least 75%, at least 80%, at least 85%, at least 90%, at least 95%, at least 96%, at least 97%, at least 98%, or at least 99% sequence identity to any one of SEQ ID:46, SEQ ID:47, and SEQ ID:48.

18. A hybridoma cell line designated as K64-8 and having an accession number of CCTCC NO: C2024342, wherein the hybridoma cell line is used to prepare the monoclonal antibody directed to a capsular polysaccharide of *K. pneumoniae* according to claim 16.

19. A monoclonal antibody directed to a capsular polysaccharide of *K. pneumoniae,* wherein the *K. pneumoniae* is K64-type *K. pneumoniae,* the antibody has a sequence comprising a light chain variable region and a heavy chain variable region, the light chain variable region has an amino acid sequence as shown in SEQ ID NO:49, or a sequence having at least 70%, at least 75%, at least 80%, at least 85%, at least 90%, at least 95%, at least 96%, at least 97%, at least 98%, or at least 99% sequence identity thereto, and the heavy chain variable region has an amino acid sequence as shown in SEQ ID NO:50, or a sequence having at least 70%, at least 75%, at least 80%, at least 85%, at least 90%, at least 95%, at least 96%, at least 97%, at least 98%, or at least 99% sequence identity thereto.

20. The monoclonal antibody directed to a capsular polysaccharide of *K. pneumoniae* according to claim 19, wherein the light chain variable region has a complementarity determining region comprising three sequences as shown in SEQ ID NO:51, SEQ ID NO:52, and SEQ ID NO:53, or a sequence having at least 70%, at least 75%, at least 80%, at least 85%, at least 90%, at least 95%, at least 96%, at least 97%, at least 98%, or at least 99% sequence identity to any one of SEQ ID NO:51, SEQ ID NO:52, and SEQ ID NO:53, and the heavy chain variable region has a complementarity determining region comprising three sequences as shown in SEQ ID:54, SEQ ID:55, and SEQ ID:56, or a sequence having at least 70%, at least 75%, at least 80%, at least 85%, at least 90%, at least 95%, at least 96%, at least 97%, at least 98%, or at least 99% sequence identity to any one of SEQ ID:54, SEQ ID:55, and SEQ ID:56.

21. A hybridoma cell line designated as K64-27 and having an accession number of CCTCC NO: C2024343, wherein the hybridoma cell line is used to prepare the monoclonal antibody directed to a capsular polysaccharide of *K. pneumoniae* according to claim 19.

22. A pharmaceutical composition, comprising the monoclonal antibody directed to a capsular polysaccharide of *K. pneumoniae* according to any one of claims 1, 4, 7, 10, 13, 16, and 19, and further comprising any one or a combination of more than one of a pharmaceutically acceptable carrier, an excipient, or a diluent.

23. A kit for detecting K64-type *K. pneumoniae,* comprising the monoclonal antibody directed to a capsular polysaccharide of *K. pneumoniae* according to any one of claims 1, 4, 7, 10, 13, 16, and 19, and further comprising any one or a combination of more than one of a positive control article, a negative control article, an antibody dilution, a chromogen solution, a stop solution, a blocking solution, or a wash solution.

24. An application of the monoclonal antibody directed to the capsular polysaccharide of *K. pneumoniae* according to any one of claims 1, 4, 7, 10, 13, 16, and 19 in preparing a therapeutic drug and/or a detection product for infectious diseases caused by K64-type *K. pneumoniae.*
